**Europäisches Patentamt**

**European Patent Office**

**Office européen des brevets**

(19)

(11) Veröffentlichungsnummer: **0 090 246**
**B1**

(12) **EUROPÄISCHE PATENTSCHRIFT**

(45) Veröffentlichungstag der Patentschrift:
14.08.85

(51) Int. Cl.⁴: **C 07 C 49/04,** C 07 C 45/28,
C 07 C 45/84

(21) Anmeldenummer: 83102485.6

(22) Anmeldetag: 14.03.83

(54) **Verfahren zur Herstellung von Pinakolon.**

(30) Priorität: 26.03.82 DE 3211306

(43) Veröffentlichungstag der Anmeldung:
05.10.83 Patentblatt 83/40

(45) Bekanntmachung des Hinweises auf die Patenterteilung:
14.08.85 Patentblatt 85/33

(84) Benannte Vertragsstaaten:
AT BE CH DE FR GB IT LI NL

(56) Entgegenhaltungen:
CHEMICAL ABSTRACTS, Band 95, Nr. 13, September
1981, Seite 633, Nr. 114804g, Columbus, Ohio, USA

(73) Patentinhaber: BAYER AG, Konzernverwaltung RP
Patentabteilung, D-5090 Leverkusen 1 Bayerwerk (DE)

(72) Erfinder: Rauleder, Gebhard, Dr., Mozartstrasse 20,
D-5657 Haan (DE)
Erfinder: Waldmann, Helmut, Dr.,
Henry-T.-von-Böttinger-Strasse 15,
D-5090 Leverkusen 1 (DE)

ACTORUM AG

## Beschreibung

Die vorliegende Erfindung betrifft ein Verfahren zur Herstellung von Pinakolon, das gemäss älterer Nomenklatur auch als Pinakolin bezeichnet wird, aus 2,3-Dimethyl-2-buten und Wasserstoffperoxid in Gegenwart von Carbonsäuren.

Pinakolon ist ein bekanntes organisches Zwischenprodukt, das insbesondere zur Herstellung von Pflanzenschutzmitteln verwendet wird (s. z. B. „Ullmanns Enzyklopädie der technischen Chemie", 4. Auflage, Bd. 14 [1977], S. 203).

In der JP-OS 5636427 (=JP-Patentanmeldung Nr. 113199 vom 3.9.1979) ist bereits ein Verfahren zur Herstellung von Pinakolon aus 2,3-Dimethyl-2-buten und Wasserstoffperoxid in Gegenwart von Carbonsäuren und starken anorganischen Säuren beschrieben worden. Wie im Beispiel dieser JP-OS näher ausgeführt wird, wurden bei 40° C zu einer Mischung, die 1,1,2,2-Tetrachlorethan, Ameisensäure, bezogen auf die Ameisensäure 6,6 Vol.-% Schwefelsäure, einen Teil der einzusetzenden Wasserstoffperoxid-Lösung und 2,3-Dimethyl-2-buten enthält, 30%iges Wasserstoffperoxid zugeführt. Anschliessend wurde auf 60° C erhitzt und weitere 3 h gerührt. Zu dieser Mischung wurden dann, bezogen auf das eingesetzte 2,3-Dimethyl-2-buten mehr als die 10fache Gewichtsmenge 27%ige Schwefelsäure gegeben und anschliessend 2 h am Rückfluss gekocht. Danach wurde die organische Phase abgetrennt, mit 5%iger Natronlauge und Wasser neutral gewaschen, mit Natriumsulfat getrocknet und destilliert. Die Ausbeute an Pinakolon betrug 64%.

Ein wesentlicher Nachteil dieses Verfahrens sind die grossen Mengen an Schwefelsäure, die benötigt werden. Weiterhin sind über die Aufarbeitung des Ameisensäure-Schwefelsäure-Wasser-Gemisches und seine Wiederverwendung keine Angaben gemacht. Eine direkte Wiederverwendung dieses Gemisches ist aber in keinem Fall möglich, da es sich sonst durch eingebrachtes und in der Reaktion gebildetes Wasser laufend verdünnen würde. Die Carbonsäure verbleibt in der Schwefelsäure-Lösung und würde sich dort anreichern. Schliesslich ist auch die Verwendung eines organischen Lösungsmittels und die damit verbundene zweiphasige Arbeitsweise nachteilig.

Es wurde nun ein Verfahren zur Herstellung von Pinakolon aus 2,3-Dimethyl-2-buten und Wasserstoffperoxid in Gegenwart von Wasser, Carbonsäuren und gegebenenfalls einer starken Säure gefunden, das dadurch gekennzeichnet ist, das man in eine Lösung, die Wasser, eine oder mehrere Carbonsäuren mit 1 bis 8 C-Atomen und gegebenenfalls kleine Mengen einer starken Säure enthält, 2,3-Dimethyl-2-buten und eine wässerige Lösung von Wasserstoffperoxid bei einer Temperatur von 20 bis 90° C zugibt, nach weitgehendem oder vollständigem Umsatz des Wasserstoffperoxids die Temperatur erhöht und das gebildete Pinakolon als azeotropes Gemisch mit Wasser abdestilliert.

Zum Einsatz in das erfindungsgemässe Verfahren geeignete Carbonsäuren sind beispielsweise Monocarbonsäuren mit 1 bis 7 C-Atomen und Dicarbonsäuren mit 3 bis 8 C-Atomen. Bevorzugt verwendet werden Ameisensäure, Essigsäure, Propionsäure, Malonsäure, Maleinsäure, Fumarsäure und Glutarsäure. Im allgemeinen wird nur eine Carbonsäure eingesetzt, es können jedoch auch mehrere Carbonsäuren eingesetzt werden.

Die Menge Wasser, die zusammen mit der oder den Carbonsäuren vorgelegt wird, kann beispielsweise so gewählt werden, dass die Konzentration der Carbonsäure(n) im Wasser 25 bis 85 Gew.-% beträgt. Vorzugsweise beträgt diese Konzentration 30 bis 60 Gew.-%.

Die Konzentration an starker Säure in der wässerigen Lösung der Carbonsäure(n) kann beispielsweise 0 bis 30 Gew.-% betragen. Beim Einsatz von starken Carbonsäuren, beispielsweise beim Einsatz von Maleinsäure oder Fumarsäure, wird das erfindungsgemässe Verfahren vorzugsweise in Abwesenheit anderer starker Säuren durchgeführt. Beim Einsatz anderer Carbonsäuren als Maleinsäure oder Fumarsäure wird die Konzentration an starker Säure vorzugsweise auf 5 bis 20 Gew.-% (bezogen auf die gesamte vorgelegte Lösung) eingestellt.

Geeignete starke Säuren sind beispielsweise Mineralsäuren und aromatische Sulfonsäuren. Vorzugsweise wird Schwefelsäure oder p-Toluolsulfonsäure eingesetzt, besonders bevorzugt Schwefelsäure.

In das Gemisch aus Wasser, Carbonsäure und gegebenenfalls starker Säure wird dann 2,3-Dimethyl-2-buten und eine wässerige Lösung von Wasserstoffperoxid zugegeben. Dabei kann das Molverhältnis von eingesetztem Wasserstoffperoxid zu Carbonsäure beispielsweise 0,3:1 bis 5:1 betragen. Vorzugsweise liegt es im Bereich 0,5:1 bis 4:1.

Das Molverhältnis von eingesetztem Wasserstoffperoxid zu eingesetztem 2,3-Dimethyl-2-buten kann beispielsweise 0,2:1 bis 1,2:1 betragen. Vorzugsweise liegt es im Bereich 0,7:1 bis 1,15:1, besonders bevorzugt zwischen 1:1 und 1,1:1.

Zum Einsatz in das erfindungsgemässe Verfahren sind wässerige Wasserstoffperoxidlösungen unterschiedlicher Konzentration geeignet, beispielsweise 30 bis 70 gew.-%ige, vorzugsweise 40 bis 60 gew.-%ige wässerige Wasserstoffperoxidlösungen.

Das erfindungsgemässe Verfahren kann während der Zugabe von Wasserstoffperoxid und 2,3-Dimethyl-2-buten bei den verschiedensten Drukken durchgeführt werden. Der Druck ist dabei vorzugsweise mindestens so hoch, dass keine nennenswerten Anteile des Reaktionsgemisches verdampfen. Die Temperatur während der Zugabe von 2,3-Dimethyl-2-buten und wässerigem Wasserstoffperoxid liegt im Bereich von 20 bis 90° C. Vorzugsweise liegt diese Temperatur im Bereich von 30 bis 65° C. Bei der bevorzugten Reaktionstemperatur kann man bei Normaldruck arbeiten. Besonders bevorzugt sind Temperaturen im Bereich von 50 bis 65° C und Normaldruck.

Als Werkstoffe für die Reaktionsapparate zur Durchführung des erfindungsgemässen Verfahrens können beispielsweise Glas, Edelstähle oder emailliertes Material verwendet werden.

Neben der Arbeitsweise unter isothermen Bedingungen, d. h. Einhaltung einer einheitlichen Temperatur während und nach der Zugabe von 2,3-Dimethyl-2-buten und wässerigem Wasserstoffperoxid, kann das erfindungsgemässe Verfahren auch unter Ausbildung eines sogenannten Temperaturgradienten durchgeführt werden, der im allgemeinen mit fortschreitender Zugabe von 2,3-Dimethyl-2-buten und wässerigem Wasserstoffperoxid zunimmt. Man kann aber auch die Reaktion so führen, dass sich mit dem Fortschreiten der Reaktion ein Gradient fallender Temperatur ausbildet.

Nach Beendigung der Zugabe von wässerigem Wasserstoffperoxid und 2,3-Dimethyl-2-buten ist es im allgemeinen erforderlich, das Reaktionsgemisch, vorzugsweise unter Rühren, noch einige Zeit auf Reaktionstemperatur zu halten, bis das Wasserstoffperoxid weitgehend oder vollständig umgesetzt ist. Sobald festgestellt wird, z. B. durch entsprechende Analyse, dass der Wasserstoffperoxidumsatz einen Wert von z. B. über 90%, vorzugsweise über 95% erreicht hat, wird die Temperatur erhöht und das gebildete Pinakolon als azeotropes Gemisch mit Wasser abdestilliert. Bei dieser Destillation können gegebenenfalls als Nebenprodukte gebildetes Aceton und/oder Dimethylbutadien mit über Kopf gehen.

Das nach dieser azeotropen Destillation zurückbleibende Gemisch, das im wesentlichen die eingesetzte Carbonsäure, Wasser und gegebenenfalls die eingesetzte starke Säure enthält, kann direkt in einen neuen Reaktionszyklus eingesetzt werden, indem man ihm erneut 2,3-Dimethyl-2-buten und wässeriges $H_2O_2$ unter den erfindungsgemässen Bedingungen zufügt.

Aus dem bei der azeotropen Destillation als Kopfprodukt anfallenden Gemisch kann man Pinakolon isolieren, indem man nach seiner Kondensation das Wasser in einem Abscheider abtrennt und aus der verbleibenden organischen Phase das Pinakolon durch Redestillation gewinnt. Die azeotrope Destillation wird im allgemeinen bei Normaldruck durchgeführt. Sie kann aber auch bei erhöhtem oder vermindertem Druck durchgeführt werden.

Ein besonderer Vorteil des erfindungsgemässen Verfahrens besteht darin, dass nach dem Abdestillieren von Pinakolon und Wasser das zurückbleibende Gemisch aus Carbonsäure, Wasser und gegebenenfalls starker Säure im Reaktor verbleiben und ohne weitere Massnahmen zu ergreifen direkt für einen neuen Ansatz verwendet werden kann. Weiterhin benötigt man für das erfindungsgemässe Verfahren im Gegensatz zum Stand der Technik kein organisches Lösungsmittel und, falls überhaupt, wesentlich geringere Mengen an starker Säure.

Es ist ausgesprochen überraschend, dass mit dem erfindungsgemässen Verfahren trotzdem Pinakolon in praktisch den gleichen Ausbeuten erhältlich ist, wie gemäss dem Stand der Technik.

*Beispiel 1:*

In einem 1-l-Doppelwandkolben mit Rührer und Destillationsaufsatz wurden 116 g Maleinsäure (1 mol) und 200 g Wasser vorgelegt und auf 65° C thermostatisiert. Unter Rühren wurden über getrennte Leitungen mit Membranpumpen innerhalb einer Zeit von 1,75 h 76 g 2,3-Dimethyl-2-buten (0,9 mol) und 68 g 50%iges, wässeriges Wasserstoffperoxid (1 mol) bei Normaldruck zudosiert. Nach Beendigung des Zudosierens wurde noch 4 h bei dieser Temperatur gerührt. Dann betrug der Wasserstoffperoxid-Umsatz 95%. Anschliessend wurde die Temperatur erhöht und das gebildete Pinakolon zusammen mit Wasser, Aceton und Dimethylbutadien bei Normaldruck abdestilliert. Dabei stellte sich eine Innentemperatur von 95 bis 102° C ein. Nach dem Abtrennen der Wasserphase wurde das organische Gemisch erneut destilliert. Dabei wurde Pinakolon in einer Ausbeute von 56 g erhalten, das entspricht 62,2% der Theorie.

*Beispiel 2:*

Die im Beispiel 1 im Reaktor verbliebene wässerige Maleinsäure wurde erneut mit den in Beispiel 1 angegebenen Mengen an 2,3-Dimethyl-2-buten und 50%igem Wasserstoffperoxid zur Reaktion gebracht und wie in Beispiel 1 beschrieben aufgearbeitet. Es resultierte Pinakolon wieder in einer Ausbeute von 62% der Theorie.

Dieser Versuchsablauf wurde insgesamt zehnmal wiederholt. Auch nach der zehnten Wiederholung blieb die Pinakolon-Ausbeute mit 61% praktisch unverändert.

*Beispiel 3:*

In einem Reaktor wie in Beispiel 1 beschrieben wurden 23 g Ameisensäure (0,5 mol), 47 g Wasser und 10 g konz. Schwefelsäure vorgelegt und auf 65° C thermostatisiert. Anschliessend wurden, wie im Beispiel 1 beschrieben, 168 g 2,3-Dimethyl-2-buten (2 mol) und 136 g 50%iges Wasserstoffperoxid (2 mol) innerhalb von 3,5 h zudosiert. Anschliessend wurde noch 3 h bei 65° C gerührt. Der $H_2O_2$-Umsatz betrug dann 98%. Dann wurde die Temperatur erhöht und die Reaktionsprodukte zusammen mit Wasser bei Normaldruck abdestilliert. Nach der Aufarbeitung wie in Beispiel 1 beschrieben resultierte Pinakolon in einer Ausbeute von 124 g, entsprechend 62% der Theorie.

*Beispiel 4:*

Zu einer Lösung von 30 g Essigsäure (0,5 mol), 50 g Wasser und 10 g konz. Schwefelsäure wurden, wie in Beispiel 1 beschrieben, bei 65° C 84 g 2,3-Dimethyl-2-buten (1 mol) und 68 g 50%iges Wasserstoffperoxid (1 mol) innerhalb von 1,75 h zudosiert. Nach 3stündigem weiterem Rühren bei 65° C betrug der Wasserstoffperoxid-Umsatz 98%. Dann wurde die Temperatur erhöht und die Reaktionsprodukte, wie im Beispiel 1 beschrieben, abdestilliert. Nach erneuter Destillation der organischen Phase wurden 120 g Pinakolon erhalten, was einer Ausbeute von 60% der Theorie entspricht.

## Patentansprüche

1. Verfahren zur Herstellung von Pinakolon aus 2,3-Dimethyl-2-buten und Wasserstoffperoxid in Gegenwart von Wasser und gegebenenfalls einer starken Säure, dadurch gekennzeichnet, dass man in eine Lösung, die Wasser, eine oder mehrere Carbonsäuren mit 1 bis 8 C-Atomen und gegebenenfalls kleine Mengen einer starken Säure enthält, 2,3-Dimethyl-2-buten und eine wässerige Lösung von Wasserstoffperoxid bei einer Temperatur von 20 bis 90° C zugibt, nach weitgehendem oder vollständigem Umsatz des Wasserstoffperoxids die Temperatur erhöht und das gebildete Pinakolon als azeotropes Gemisch mit Wasser abdestilliert.

2. Verfahren nach Anspruch 1, dadurch gekennzeichnet, dass man als Carbonsäure eine Monocarbonsäure mit 1 bis 7 C-Atomen oder eine Dicarbonsäure mit 3 bis 8 C-Atomen einsetzt.

3. Verfahren nach Ansprüchen 1 und 2, dadurch gekennzeichnet, dass man eine 25 bis 85 gew.-%ige wässerige Carbonsäurelösung einsetzt.

4. Verfahren nach Ansprüchen 1 bis 3, dadurch gekennzeichnet, dass die Konzentration an starker Säure in der Wasser und Carbonsäure enthaltenden Lösung 0 bis 30 Gew.-% beträgt.

5. Verfahren nach Ansprüchen 1 bis 4, dadurch gekennzeichnet, dass das Molverhältnis des eingesetzten Wasserstoffperoxids zu Carbonsäure 0,3:1 bis 5:1 beträgt.

6. Verfahren nach Ansprüchen 1 bis 5, dadurch gekennzeichnet, dass das Molverhältnis von eingesetztem Wasserstoffperoxid zu eingesetztem 2,3-Dimethyl-2-buten 0,2:1 bis 1,2:1 beträgt.

7. Verfahren nach Ansprüchen 1 bis 6, dadurch gekennzeichnet, dass man die Zugabe von Wasserstoffperoxid und 2,3-Dimethyl-2-buten bei Temperaturen im Bereich 30 bis 65° C und bei Normaldruck vornimmt.

8. Verfahren nach Ansprüchen 1 bis 7, dadurch gekennzeichnet, dass man nach Beendigung der Zugabe von Wasserstoffperoxid und 2,3-Dimethyl-2-buten das Reaktionsgemisch so lange auf Reaktionstemperatur hält, bis der Wasserstoffperoxidumsatz über 90% beträgt.

9. Verfahren nach Ansprüchen 1 bis 8, dadurch gekennzeichnet, dass man das gebildete Pinakolon als azeotropes Gemisch mit Wasser bei Normaldruck abdestilliert.

10. Verfahren nach Ansprüchen 1 bis 9, dadurch gekennzeichnet, dass man bei der azeotropen Destillation das Kopfprodukt kondensiert, in einem Abscheider das Wasser abtrennt und aus der verbleibenden organischen Phase das Pinakolon durch Redestillation gewinnt.

## Claims

1. Process for preparing pinacolone from 2,3-dimethyl-2-butene and hydrogen peroxide in the presence of water and, if desired, a strong acid, characterised in that 2,3-dimethyl-2-butene and an aqueous solution of hydrogen peroxide are added at a temperature of 20 to 90° C to a solution which contains water and one or more carboxylic acids having 1 to 8 C atoms and which can, if desired, also contain small amounts of a strong acid, the temperature is increased after substantial or complete reaction of the hydrogen peroxide, and the pinacolone formed is distilled off as an azeotropic mixture with water.

2. Process according to Claim 1, characterised in that the carboxylic acid used is a monocarboxylic acid having 1 to 7 C atoms or a dicarboxylic acid having 3 to 8 C atoms.

3. Process according to Claims 1 and 2, characterised in that a 25 to 85% by weight aqueous carboxylic acid solution is used.

4. Process according to Claims 1 to 3, characterised in that the concentration of strong acid in the solution containing water and carboxylic acid is 0 to 30% by weight.

5. Process according to Claims 1 to 4, characterised in that the molar ratio of hydrogen peroxide used to carboxylic acid is 0.3:1 to 5:1.

6. Process according to Claims 1 to 5, characterised in that the molar ratio of hydrogen peroxide used to 2,3-dimethyl-2-butene used is 0.2:1 to 1.2:1.

7. Process according to Claims 1 to 6, characterised in that hydrogen peroxide and 2,3-dimethyl-2-butene are added at temperatures within a range of 30 to 65° C and under normal pressure.

8. Process according to Claims 1 to 7, characterised in that after the addition of hydrogen peroxide and 2,3-dimethyl-2-butene is complete the reaction mixture is maintained at the reaction temperature until the degree of hydrogen peroxide conversion is above 90%.

9. Process according to Claims 1 to 8, characterised in that the pinacolone formed is distilled off under normal pressure as an azeotropic mixture with water.

10. Process according to Claims 1 to 9, characterised in that, in the azeotropic distillation, the top product is condensed, water is separated off in a water separator and pinacolone is obtained by redistillation from the remaining organic phase.

## Revendications

1. Procédé de fabrication de pinacolone à partir de 2,3-diméthyl-2-butène et de peroxyde d'hydrogène en présence d'eau et éventuellement d'un acide fort, caractérisé en ce que, dans une solution qui contient de l'eau, un ou plusieurs acides carboxyliques ayant 1 à 8 atomes de carbone et éventuellement de petites quantités d'un acide fort, on ajoute du 2,3-diméthyl-3-butène et une solution aqueuse de peroxyde d'hydrogène à une température de 20 à 90° C, on augmente la température après conversion substantielle ou totale de peroxyde d'hydrogène et on sépare par distillation la pinacolone formée sous la forme d'un mélange azéotropique avec l'eau.

2. Procédé selon la revendication 1, caractérisé en ce qu'on utilise comme acide carboxylique un acide monocarboxylique ayant 1 à 7 atomes de carbone ou un acide dicarboxylique ayant 3 à 8 atomes de carbone.

3. Procédé selon les revendications 1 et 2, caractérisé en ce qu'on utilise une solution aqueuse d'acide carboxylique à 25-85% en poids.

4. Procédé selon les revendications 1 à 3, caractérisé en ce que la concentration en acide fort dans la solution contenant de l'eau et l'acide carboxylique s'élève à 0-30% en poids.

5. Procédé selon les revendications 1 à 4, caractérisé en ce que le rapport molaire du peroxyde d'hydrogène employé envers l'acide carboxylique est de 0,3:1 à 5:1.

6. Procédé selon les revendications 1 à 5, caractérisé en ce que le rapport molaire du peroxyde d'hydrogène employé envers le 2,3-diméthyl-2-butène s'élève à 0,2:1 jusqu'à 1,2:1.

7. Procédé selon les revendications 1 à 6, caractérisé en ce qu'on entreprend l'addition du peroxyde d'hydrogène et du 2,3-diméthyl-2-butène à des températures dans l'intervalle de 30 à 65°C sous la pression normale.

8. Procédé selon les revendications 1 à 7, caractérisé en ce qu'au terme de l'addition du peroxyde d'hydrogène et du 2,3-diméthyl-2-butène on maintient le mélange de réaction à la température de réaction jusqu'à ce que la conversion du peroxyde d'hydrogène s'élève à plus de 90%.

9. Procédé selon les revendications 1 à 8, caractérisé en ce qu'on sépare par distillation la pinacolone formée sous forme de mélange azéotropique avec de l'eau sous la pression normale.

10. Procédé selon les revendications 1 à 9, caractérisé en ce qu'on condense dans la distillation azéotropique le produit de tête, on sépare l'eau dans un séparateur et l'on récupère à partir de la phase organique restante la pinacolone par une redistillation.